# EUROPEAN PATENT SPECIFICATION

(11) **EP 0 778 049 B1**
(45) Date of publication and mention of the grant of the patent: **18.02.2004**
(21) Application number: 96119346.3
(22) Date of filing: 03.12.1996
(51) Int. Cl.: A61N 1/365

(54) **A heart pacemaker with a variable stimulation frequency**
Herzschrittmacher mit veränderbarer Reizungsfrequenz
Stimulateur cardiaque à fréquence de stimulation variable

(30) Priority: 05.12.1995 IT TO950968
(43) Date of publication of application: 11.06.1997
(73) Proprietor: SORIN BIOMEDICA CRM S.r.l., 13040 Saluggia (Vercelli) (IT)
(72) Inventor: Plicchi, Gianni, 40136 Bologna (IT); Corbucci, Giorgio, 40017 San Giovanni in Persiceto(Bologna) (IT); Garberoglio, Bruno, 10156 Torino (IT)
(74) Representative: Bosotti, Luciano

(56) References cited:
- US-A- 4 428 378
- US-A- 5 031 614
- US-A- 5 383 911

## Description

### Field of the invention

The present invention relates to the field of electrical heart-stimulation devices such as, for example, adaptive heart pacemakers, in which the stimulation frequency is determined by the patient's physical activity; this can be detected by a suitable sensor located inside the casing of the heart pacemaker, stuck to its walls or fitted as a component in the electronic circuit.

This particular type of adaptive heart pacemaker is defined as "activity-controlled" and is currently the most widespread in the absolute sense with more than 350 thousand implants worldwide.

### Description of the prior art

There are essentially three types of activity-controlled pacemaker currently in use, all being.characterized by the ability to detect movements and vibrations which are transmitted from the patient to the implanted pacemaker.

The three most widespread types of activity-controlled heart pacemaker are distinguished mainly by the type of sensor used, whilst each type using the same type of sensor may include a subgroup of products which use the same type of sensor but analyze the same signal by different techniques, processing specific and personalized algorithms for determining the stimulation frequency.

The prior art in this field will be analyzed briefly below, each type of sensor and any subgroups of products being associated, as far as they are known, with the differentiating features drawn from publications and published patents on the subject.

The three types are characterized by three specific types of activity sensor: electro-mechanical sensors, piezoelectric sensors, and accelerometric sensors.

Electro-mechanical sensors are constituted by small devices in which movement brings about macroscopic displacement or deformation of mechanical masses, magnetized microspheres, or droplets of mercury, which induce corresponding electrical signals, either by variation of the magnetic field or by the closure of electrical contacts.

In particular, a magnetized microsphere which moves freely and generates a movement-dependent signal in surrounding inductances is used in the Sensorithm pacemaker produced by Siemens, whereas a pacemaker dependent upon the deformations of a droplet of mercury is described in EP-A-0 383 732 and is used in Sorin Biomedica Cardio S.p.A.'s Swing pacemakers.

Other movement sensors which operate by the opening and closure of contacts are described in US-A-4 771 780 in the name of Siemens-Pacesetter and in the publication "A new mechanical sensor for detecting body activity and posture, suitable for rate responsive pacing" by Alt E., Matula M., Thilo R., et al. in PACE 1988 pp. 11:1875-1881.

Owing to their nature, electro-mechanical sensors are sensitive to any type of movement which alters the equilibrium position of the movable mass, whereas they are relatively insensitive to vibrations which, by definition, are constituted by small movements which are symmetrical about an equilibrium point.

A piezoelectric sensor is generally constituted by a linear element of thin piezoelectric material which is glued to the internal wall of the pacemaker casing in order to transduce into an electrical signal vibrations which the human body may produce in the course of active movement or to which it may be subjected passively by the environment with which it is in contact, for example, when travelling in a means of transport.

The application of this principle to activity-controlled adaptive heart pacemakers is described in US-A-4 428 378 in the name of Medtronic Inc. and has been used widely in many of that company's commercial pacemaker models such as, for example, Activitrax and Legend. In this case, the processing of the signal consists in the detection of the frequency of the peaks of the signal, upon which the stimulation frequency is made dependent; it has in fact been found that, if the activity level increases, the spectral content of the signal generated by the sensor translates incrementally in the 1-20 Hz band and there is a parallel increase in the frequency of the peaks detected.

Other applications use exactly the same sensor but the type of signal processing and the algorithm which associates it with the stimulation frequency are changed.

In particular, the article "ERGOS" - Rate Adaptive Pacing System based on Motion Energy" by M. Schaldach, published in Biotronik Review, Vol. 1:1 of 1989 describes how, in Biotronik pacemaker models Ergos 1 and Ergos 2, after pass-band filtering centred on 4 Hz, the signal of the piezoelectric sensor no longer uses the frequency of the peaks beyond a certain threshold as a useful signal for determining the stimulation frequency, as described in US-A-4 428 378 already cited, but uses the time at which the signal exceeds a certain threshold; according to the author, the control signal obtained is thus determined to a greater extent by the energy expended by the patient for the movement.

In US-A-4 940 053 in the name of Siemens Pacesetter Inc., which relates to the Sensolog pacemaker produced by that Company, the signal generated by an identical piezoelectric sensor is rectified and integrated in order to derive the energy content and to use it to determine the stimulation frequency in a linear relationship.

Accelerometric sensors are generally constituted by one or more mono-axial accelerometers which, it is asserted, are inserted as components in the electronic circuit and are thus not in direct mechanical contact with the internal wall of the pacemaker casing. This is in order to detect solely inertial accelerations resulting from movement and to be insensitive to vibrations at frequencies greater than 10 Hz which are transmitted by the human body to the pacemaker and are interpreted as interference but which, in the piezoelectric sensors described above, constitute the useful signal.

The foregoing is fully explained in the text "Rate Adaptive Cardiac Pacing" by Chu-Pak Lau, Futura Publishing Company, Chap. 7, page 78.

EP-A-0 259 658 in the name of Intermedics Inc., describes an accelerometric sensor operating in the range of 0.3-4 Hz, in which the stimulation frequency is determined by the integral of the rectified signal. This principle is applied in the Relay and Dash models.

In the above-mentioned European document, and in the corresponding US-A-5 031 614, after which the preamble of Claim 1 was patterned, it is clearly shown in the text and in the drawings that vibrations at frequencies greater than 4 Hz are rejected and that, if these vibrations are not suppressed by filtering, they may affect the good operation of the device.

EP-A-0 495 293 in the name of Cardiac Pacemakers Inc., describes a pacemaker operating with a piezoresistive accelerometer which operates at low frequency and in which the stimulation frequency is determined by the amplitude of the low-frequency electronically-filtered signal, but there is no noticeable conceptually-significant difference with respect to EP-A-0 259 658, except for the way in which the electronic circuitry is formed.

A conceptually more complex solution is described in EP-A-0 550 293 in the name of Ela Medical, which describes a heart pacemaker in which the stimulation frequency is derived from an analysis of the signals of three accelerometers arranged perpendicular to one another, with the purpose of obtaining a complex signal. In contrast to the applications described above, in which the acceleration was evaluated along only one axis, for example, from front to back, the value of the signal is independent of the direction of movement.

It is also noted that, in this application, the accelerometer is used in a well-defined frequency range of 0.7-6 Hz in order to eliminate artifacts which occur at higher frequencies.

It can be inferred from an examination of the documentation cited that the three categories of activity sensors mentioned, that is, electro-mechanical sensors, piezoelectric sensors and accelerometric sensors, operate in substantially differentiated ways in interpreting the inertial and vibratory signals which reach the implanted heart pacemaker casing through the human body.

Electro-mechanical sensors provide signals related simply to the patient's movement and also to the absolute position of the sensor relative to the gravitational field. They show an intrinsic insensitivity to vibrations, understood as small oscillations at frequencies greater than 10 Hz.

Piezoelectric sensors detect essentially variations in deformation and, since they have large surface areas and high sensitivity and are glued to the walls of the pacemaker casing, show great sensitivity to the vibrations which occur in the course of physical activity. As such they are used to determine the stimulation frequency.

It is asserted that one- and three-dimensional accelerometers are inserted as components in the electronic circuit, mechanically isolated from the wall of the casing. This is with the declared purpose of reducing problems due to the direct transmission to the pacemaker of stresses such as, for example, impacts or pressure through the skin and they operate with the purpose of detecting inertial accelerations due to movement and characterized by a frequency band of about 1-5 Hz.

### Objects and summary of the invention

Each of the methods described offers advantages and limitations which are discussed at great length in the texts cited; however, no method solves the basic problem connected with the fact that the inertial and vibratory stresses received by the pacemaker are not always of a physiological nature.

The present invention, which has the characteristics recited in Claim 1, achieves the object of overcoming these limitations, solving the problem thus defined.

In this connection, researches carried out in the existing literature and the data obtained by the Applicant's own research confirm that the energy cost of ambulation can be linked to step frequency and to the type of ambulation, which can be identified by the impact of the foot on the ground.

Since ambulation is the physical activity which is considered the most suitable and most indicative of the metabolic needs of the wearers of heart pacemakers, the currently-preferred embodiment of the invention provides for the use of a normal accelerometric sensor located inside the heart pacemaker. This has the sole purpose of detecting the execution of each individual step and the characteristics of the impact of the foot on the ground in order to derive the step frequency and to classify the type of impact in order to determine the stimulation frequency.

Specifically, in order effectively to detect each individual step in order to calculate the step frequency and the type of impact on the ground, the currently-preferred embodiment provides for the use of a conventional micro-accelerometer (for example a piezoelectric accelerometer) which can operate in the band of about 0.1-100Hz.

This accelerometer is located inside the pacemaker so that it can have mechanical continuity with the walls of the pacemaker so as to be able to transmit undamped inertial and vibrational components in the envisaged frequency band of 0.1-100 Hz.

In practice, the accelerometer must be able to receive, and consequently transduce into signals, both of the mechanical stresses (inertial and vibratory) which currently affect piezoelectric activity sensors and accelerometers in the methods known and described up to now.

There are various possible ways of achieving a mechanical assembly which can ensure the above-mentioned undamped transmission of vibrations: by way of example, the accelerometer may be located in a manner such that it is in direct mechanical contact with one of the metal walls upon completion of the construction of the pacemaker, and/or the separation elements between the electronic circuits and the casing may be made of materials which, although electrically-insulating, have mechanical stiffness and surface-contact characteristics suitable for transmitting vibrations from the casing to the substrate of the electronic circuit, on which the accelerometer is fitted.

It will be noted that this method is diametrally opposed to that commonly adopted by other constructors who use accelerometers in which, in contrast, great efforts are made to isolate the accelerometer from the walls of the casing in order to reduce the transmission of vibrations of frequencies greater than 10 Hz which, in that case, are considered as interfering elements and artifacts.

From this point of view, the invention is, in fact, based upon the concept that the execution of a step gives rise to a low-frequency inertial acceleration linked to the displacement of the body and to high-frequency vibrations connected with the impact of the foot on the ground.

The characteristics of the two, low- and high-frequency signals which characterize the step change according to the type of ambulation so that, from a knowledge of both and from their interaction, it is possible to know with reasonable certainty that a step has been taken and the type of step, such as running, going down stairs, or walking at different speeds,with discrimination of interference and other kinds of artifacts such as, for example, stresses directed to the pacemaker through the skin, and transportation on mechanical means.

### Description of the drawings

The invention will now be described, purely by way of non-limiting example, with reference to the appended drawings, in which:
Figure 1 is a graph showing the physiological relationship between step frequency and the energy cost of ambulation,
Figure 2, which is composed of three graphs disposed one above another and indicated a), b) and c), shows qualitatively an example of a signal coming from the movement sensor of a device according to the invention and the respective low- and high-frequency components,
Figure 3 is a block diagram of the principal functions which characterize the sensor, the processing of the data, and the associated stimulation, in a pacemaker implementing the invention, and
Figure 4 shows an embodiment of a circuit layout which can derive the data relating to frequency and step type from an accelerometric signal.

### Theoretical and experimental basis of the invention

As a premise to the detailed description of an embodiment of the invention, it seems useful to describe some experimental results obtained by the Applicant.

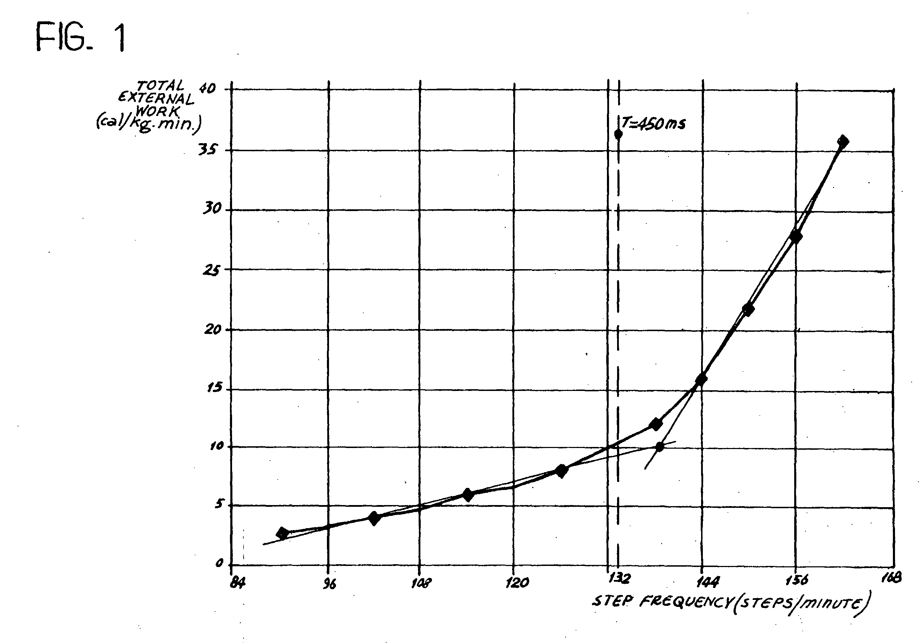

The graph of Figure 1 shows that a good correspondence can be found experimentally between step frequency (steps/minute, on the abscissa scale) and total external work (cal/kg.minute, on the ordinate, where 1 cal/kg.min corresponds to 4.1868 J/kg.min, and hence the metabolic requirement of an ambulatory human subject. There is quite a clear distinction between normal ambulation conditions (normal walking with a step period greater than about 450 ms - equal to about 132 steps/minute - shown on the left-hand side of the drawing) and running (on the right-hand side of the drawing).

The signal (the movement signal) generated by an accelerometer located inside a pacemaker fitted in a wearer was analyzed. This was to ensure undamped transmission of stresses to the accelerometer according to the criteria described above.

The wide-band signal (0.1-100 Hz) generated by the accelerometer during the wearer's movement was amplified and separated into two components in the 1-5 Hz band to identify inertial accelerations, and in the 20-50 Hz band to identify vibrations, respectively.

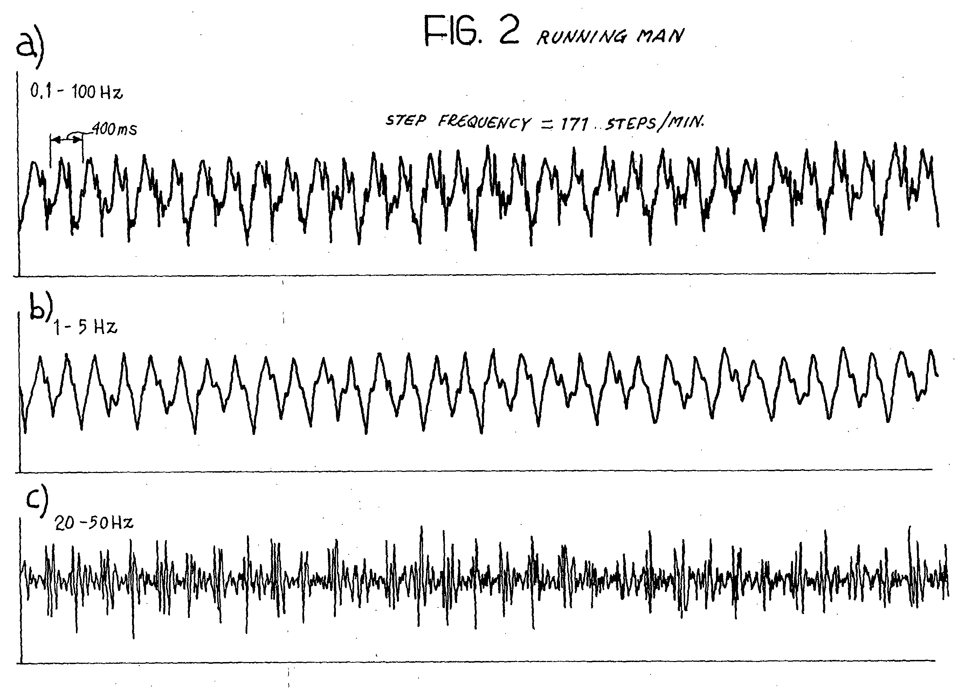

The graphs of Figure 2 show typical traces of the full accelerometric signal (graph a) and of the low-frequency (1-5 Hz - graph b) and high-frequency (20-50 Hz - graph c) components, respectively, detected in a running man (ambulation at 171 steps/minute).

For both channels, two tests of event duration and repetition period, respectively, were carried out. This was with the primary intention of identifying from the two respective inertial and vibrational components of the movement signal:
- the step frequency, and
- the type of ambulation (walking, running, etc..) identified primarily by the way in which the feet strike the ground.

The tests carried out related to both low-frequency (1-5 Hz) and high-frequency (20-50 Hz) channels, respectively.

In particular, two tests were carried out for the events detected independently in each channel: a duration test and a repetition-period test. For the low-frequency channel, the comparison duration was 10 ms, whereas for the high-frequency channel a value of 5 ms was selected. The comparison period was 300 ms for the high-frequency channel and 380 ms for the low-frequency channel.

The result of each test assigned the value "0" or "1" to the variables A, B, C, D, defined as follows:

| | |
|---|---|
| if the duration of the low-frequency event > 10ms: | A = 1 |
| otherwise: | A = 0 |
| | |
| if the repetition period of the low-frequency events > 380 ms: | B = 1 |
| otherwise | B = 0 |
| | |
| if the duration of the high-frequency event > 5ms: | C = 1 |
| otherwise | C = 0 |
| | |
| if the repetition period of the high-frequency event > 300 ms: | D = 1 |
| otherwise | D = 0. |

The limits of 5, 10, 300, 380 ms set were derived from data collected by the authors which show congruence with the execution of steps in different forms of ambulation.

However, these values should not be interpreted as limiting of the scope of the invention. In clinical practice, step periods below 300 ms have not been noted, whereas for periods below 450 ms, a considerable increase in the energy cost of ambulation is noted. Running can be associated, for example, with a period of the order of 350 ms with the simultaneous presence of low- and high-frequency components.

A combinatorial decision table which receives the values of the variables A, B, C, D as inputs can recognize steps and types of ambulation, distinguishing them from artifacts.

Table 1 below gives the combinations of the values of A, B, C, D which correspond to actual situations in which a step is present, it being understood that values of the A, B, C, D combinations not included in the table (except for the combination 0, 0, 0, 0, identifying rest conditions, which will be mentioned below) correspond to unclassifiable situations of ambulation (that is, in practice, to operating errors) and artifacts. It is also pointed out that, in the table, "1" means that the test has been passed, whereas the opposite situation is indicated by "0".

**Table 1**

| A | B | C | D |
|---|---|---|---|
| 0 | 0 | 1 | 1 |
| 1 | 0 | 1 | 1 |
| 1 | 1 | 0 | 0 |
| 1 | 1 | 1 | 0 |
| 1 | 1 | 1 | 1 |

In other words, it can be seen that of the 2⁴ = 16 hypothetically possible combinations, only a small number can in fact be considered significant.

The true steps of which the frequency is to be computed can thus be distinguished by the table in order to associate the frequency with the stimulation frequency by a suitable algorithm.

In this connection, in the situations defined as 0011, 1011, 1111 in the table, a subsequent analysis of the repetition period of the step carried out on the high-frequency signal enables the type of activity to be identified in these cases and, specifically, if T is the step repetition period, T < 450 ms indicates running whereas T > 450 ms indicates going downstairs.

The recognition of an individual step, of the type of ambulation, of the complete absence of a signal (A,B,C,D = 0,0,0,0) or of artifacts inferred from the analysis carried out by the blocks described above can thus be transmitted to the control unit of the pacemaker which provides for differentiated stimulation modes (for example, four stimulation-frequency ranges between the minimum rest frequency and the highest frequency), which are usually at least partially programmable by the operator.

The control unit recognizes the region in which the stimulation frequency is currently located and, with a suitable time constant, increases or decreases it relative to its current value and in relation to the ambulation conditions, that is, to the step frequency and to the type of ambulation (or artifacts) recognized as indicated above.

The data resulting from this comparison are interpreted at the level of the stimulation-signal generating unit, for example, as an increase in the stimulation frequency in the case of running and as a decrease in the case of going down stairs, relative to the stimulation frequency calculated during normal walking, in accordance with the physiology which shows an increase and a decrease in the energy cost of ambulation in the two situations mentioned above, respectively, when compared with ordinary walking.

### Detailed description of an embodiment of the invention

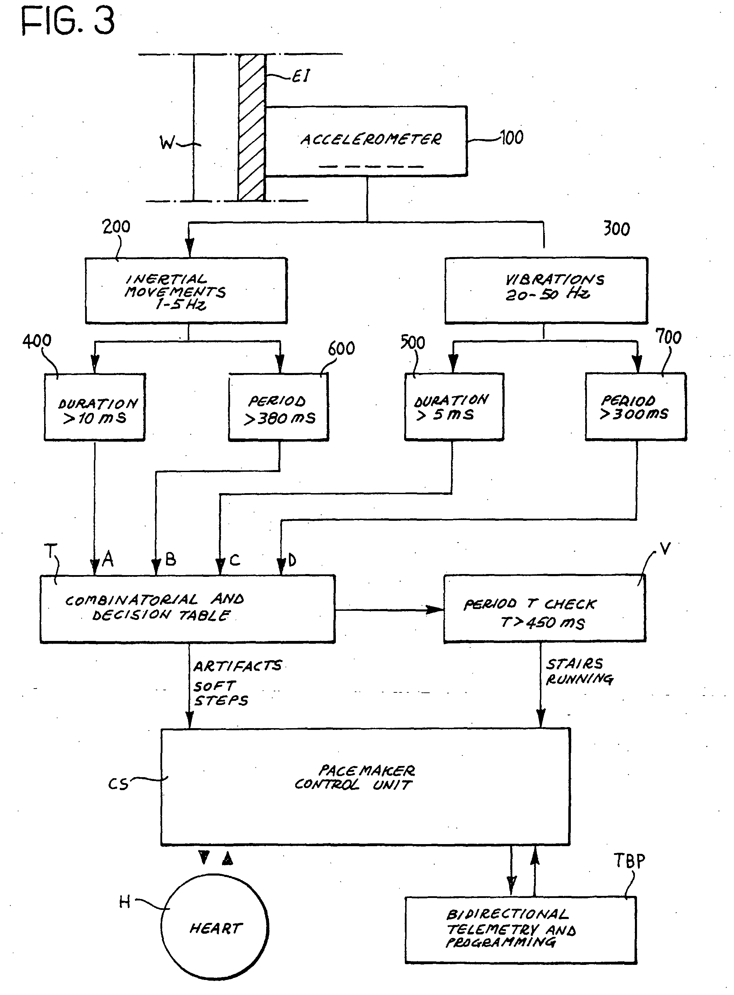

The block diagram of Figure 3 shows, at the functional level, the criteria which, in an embodiment of the pacemaker according to the invention, regulate the processing of the movement signal coming from the accelerometer 1 and its use for piloting the stimulation-control unit CS of a pacemaker for electrically stimulating a wearer's heart H. The criteria for the production of the control unit CS, which usually also has an associated bidirectional telemetry and programming function TBP, are of known type not requiring specific description herein and, furthermore, are not essential *per se* for the purposes of an understanding of the implementation of the invention.

As already stated, the accelerometer ACC must be able to receive and consequently transduce into electrical signals both of the mechanical stresses (inertial and vibrational stresses) which affect it.

There are various possible ways of achieving a mechanical assembly which can ensure the aforementioned undamped transmission of vibrations. By way of example, Figure 3 shows schematically a location of the accelerometer such that, upon completion of the construction of the pacemaker, it is disposed (for example, inside the pacemaker) in mechanical contact with one of the walls W directly or with the interposition of elements EI which separate the electronic circuits and the container and which are made of materials which, although electrically-insulating, have mechanical stiffness and surface-contact characteristics suitable for transmitting vibrations from the casing to the substrate of the electronic circuit in which the accelerometer is connected.

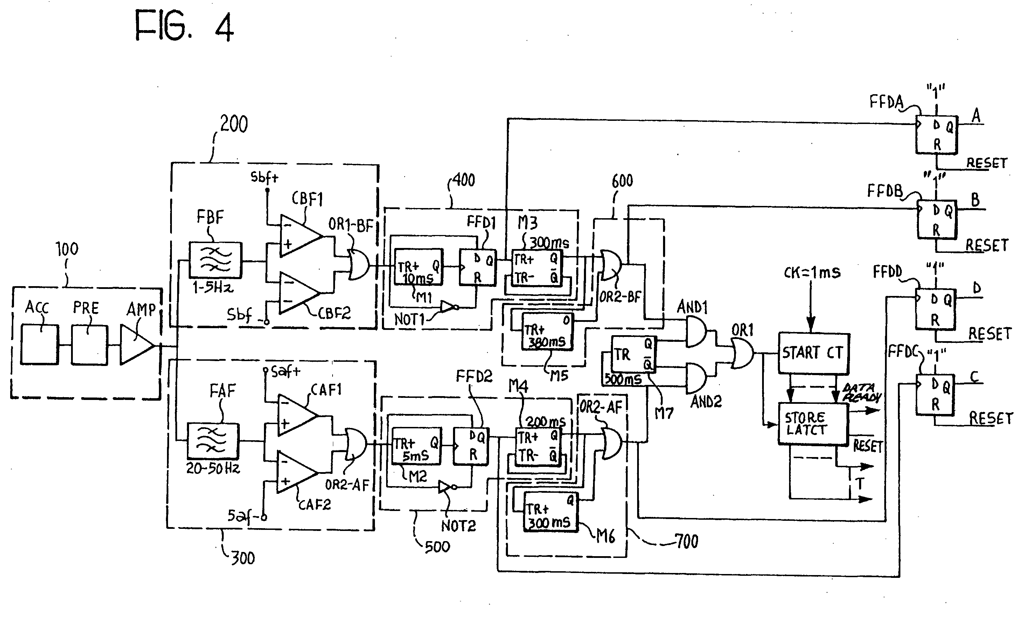

The diagram of Figure 4 shows in detail a possible embodiment of a circuit for some of the functions indicated in Figure 3. The general reference numerals indicated in Figure 3 are in any case given in Figure 4.

As stated, the present invention provides an adaptive pacemaker which can modify the stimulation frequency in dependence on the frequency or type of the wearer's steps.

In the currently-preferred embodiment, the invention provides for the use of a piezoelectric micro-accelerometer ACC having a substantially flat frequency response, for example, in the band of from 0.1-100 Hz as the movement sensor which generates the signal used for the adaptive piloting of the frequency of the electrical stimulation signal.

The fact that the sensor is characterized by this frequency band ensures an absence of distortions induced by the sensor on the low- and high-frequency components which are of interest. The possible use of movement sensors produced by different technologies, such as, for example, piezoresistive or capacitive sensors does not involve any alteration of the proposed method since it usually only involves the adjustment of any preamplification device PRE which is closely dependent upon the production technology of the accelerometer used.

In particular, for piezoelectric accelerometers, the preamplifier PRE consists simply of an impedance adaptor having the purpose of supplying an electrical signal which has low impedance and can therefore be processed by the devices in cascade therewith.

In fact, piezoelectric sensors are characterized by high output impedances (in the Gohm range) so that it would be difficult to use them without an impedance adaptor. Preamplifiers for piezoelectric sensors are typically formed by MOS transistors or operational amplifiers suitably connected to the sensor.

The actual amplification (amplifier AMP) of the movement signal and its subsequent division into first and second signals which express the two basic components, that is, the low-frequency 1-5 Hz component (FBF - low-frequency filter) and the high-frequency, 20-50 Hz component (FAF - high-frequency filter) are carried out downstream of the preamplifier PRE. The first, low-frequency, 1-5 Hz, signal detects inertial accelerations connected with the patient's movement and is digitized by a window comparator constituted by two comparators CBF1 (a positive-threshold low-frequency comparator) and CBF2 (a negative-threshold low-frequency comparator) with symmetrical thresholds, and by an OR logic gate OR1-BF which transforms the signals crossing the positive and negative thresholds Sbf+ and Sbf- into digital pulses of which exclusively a time analysis is carried out by the following stages. The thresholds Sbf+ and Sbf- are very low in order to detect even movements of small magnitude.

The second, high-frequency, 20-50 Hz, signal detects vibrations transmitted by the patient's body to the pacemaker and thus provides information on the type of impact of his foot on the ground. This signal is also digitized by a window comparator constituted by two comparators CAF1 (a positive-threshold high-frequency comparator) and CAF2 (a negative-threshold high-frequency comparator) with symmetrical thresholds, and by an OR logic gate OR1-AF which transform all of the signals which pass over the thresholds Saf+ and Saf- into digital pulses.

Clearly, the terms "low-frequency" and "high-frequency" should be interpreted as relative to one another, with general reference to the typical frequency range (by way of indication 0-100 Hz) of a movement sensor for pacemakers. The first test carried out on both first and second signals, that is, the low- and high-frequency signals, respectively, relates to the durations of the individual events detected by the window comparators.

For the first, low-frequency signal, the duration must be greater than 10 ms, whereas for the second, high-frequency signal, the duration has to be greater than 5 ms. In the diagram of Figure 4, the circuit which performs the duration test for the low-frequency signal is constituted by a retriggerable monostable unit M1 (10 ms), by an invertor NOT1 and by a D-type Flip-Flop FFD1; the output Q of FFD1 becomes high only when there are signals with durations > 10 ms.

Similarly, the circuit which performs the duration test on the digitized high-frequency signal is constituted by retriggerable monostable unit M2 (5 ms), by an inverter NOT2, and by a D-type Flip-Flop FFD2; the output of FFD2 becomes high only for signals with durations > 5 ms. However, more than one pulse may be produced for each event; a typical situation is that in which the output of the window comparator generates a set of two pulses for one step.

For this reason, two non-retriggerable monostable units, M3, 300 ms, and M4, 200 ms, respectively, which are sensitive only to the first pulse which passes the test, are used in cascade with the two Flip-Flops FFD1 and FFD2 connected to the two window comparators of the low- and high-frequency chains. The result is the exclusion of multiple pulses, in the sense that the set of pulses relating to a single step is transformed into a single pulse of fixed duration representing the step. These same signals input to monostable units M3 and M4 set, respectively, the variables "A" and "C" indicating the presence of low and high-frequency signals which have passed the first test, that is, the duration test. The memories which hold the values of the variables "A" and "C" are D-type Flip-Flops FFDA and FFDC, respectively, the inputs D of which are kept fixed at the logic level "1".

A test of the repetitiveness of the events detected is then carried out for both processing chains; the steps must have a repetition period no less than 300 ms. However, as far as the low-frequency signals are concerned, it is important for M3 to have a period equal to 300 ms to ensure that it includes all of any pulses associated with a single step; however, this may render critical the subsequent check on the repetition period which is also 300 ms. It should at this point be noted that step periods of the order of 400 ms relate to running and no longer to normal walking and, in running, the impact on the ground is in any case such as always to generate high, high-frequency signals, which enable a safe evaluation to be made irrespective of what takes place in the low-frequency channel. It should also be stressed that fast walking which is not such as to generate appreciable high-frequency signals never falls below 450 ms. A test on the repetition period of the low-frequency signal is therefore performed with a period of 380 ms as a reference: if there are actual step signals with a period below 380 ms they are in any case detected by the high-frequency channel since they relate to running and the corresponding configuration would be A,B,C,D, = 1,0,1,1. If, on the other hand there are signals which have a repetition period below 380 ms but are present only on the low-frequency channel, they have nothing to do with running but are probably connected with artifacts generated mechanically, for example, by alternating and frequent pressure on the pacemaker through the skin and without impacts. If, however, there is a step with a period of about 400 ms, the configuration will be A,B,C,D = 1,1,1,1 which, like A,B,C,D = 1,0,1,1 already mentioned, corresponds to running.

The period test is carried out by means of a retriggerable monostable unit M5 (380 ms) for'the low-frequency chain and M6 (300 ms) for the high-frequency chain and an OR logic gate OR2-BF for the low-frequency and OR2-AF for the high-frequency, which prevent the passage of signals which, although they have passed the first test relating to duration do not pass the second.

The outputs of OR2-BF and OR2-AF set the variables "B" and "D" indicating the passing of the repetition-period test by the low and high-frequency components, respectively. The memories which hold the values of the variables "B" and "D" are also D-type Flip- Flops FFDB and FFDD, respectively, the inputs D of which are kept at "1".

The signals A, B, C, D are thus set by the edges of their control signals and not by the respective logic levels; this ensures correct detection of the noise situation corresponding to signals which repeat with periods < 300 ms for the high frequency and < 380 ms for the low frequency.

In this situation M6 and M5 would always have "high" outputs but this does not affect B and D which switch only in the presence of leading edges.

At this point, the circuit can supply the variables A, B, C, D to a table decision function (usually implemented at the level of the microprocessor which is normally present in the unit CS, although in Figure 3 it is represented separately at T for purposes of clarity). These variables enable the control unit CS of the pacemaker to perform a first classification of the signals generated by the accelerometer. Since, as stated, the combinatorial decision table T is usually implicitly inside the control unit of the pacemaker, the unit in question reads the variables A,B,C,D, cyclically and zeros them after reading, evaluating their combinations and thus classifying the signal detected by the accelerometer.

The configuration A,B,C,D = 0,0,0,0 indicates a complete absence of signal and, in this situation, the control unit of the pacemaker reduces the stimulation frequency for each heart cycle down to the lower limit programmed and always in compliance with the time constants programmed.

If A,B,C,D, = 1,1,0,0-1,1,1,0, the patient is walking normally since the low-frequency components of the signal generated by the accelerometer (the first signal) are present and pass both tests but the high-frequency components (the second signal) are not present or are not reliably present; the frequency of the steps in question will enable the control unit of the pacemaker to calculate the corresponding stimulation frequency, in compliance with the time constants programmed.

Configurations including B = 1 when A = 0 and, correspondingly D = 1 when C = 0 are not permitted according to the electronic layout proposed, since the signals which do not pass the first duration test are not allowed through and are not therefore subjected to the second test relating to the repetition period.

Configurations including C,D = 1,1 irrespective of the value of A and B correspond to high-frequency signals which have passed both initial duration and repetitiveness tests and relate to steps characterized by a heavy impact on the ground; this means that running or going down stairs may be involved.

An analysis of the period T (function V of Figure 3) may be used to clarify this; if T > 450 ms the step detected is classified as going down stairs, for T < 450 ms, it is classified as running.

If running is detected, the control unit CS of the pacemaker reduces the time constant which regulates the increase in stimulation frequency, enabling the stimulation frequency to be adjusted more quickly to high values corresponding to the patient's needs. If going down stairs is detected, the time constant relating to the increase in heart rate is increased in order to limit the increase in the stimulation frequency relative to the minimum value in a situation which involves a slight effort, generally of short duration.

In this connection, it should be stated that, as is well known to experts in the art, the stimulation-control strategy may differ according to specific requirements of use and consequent design selections.

The unit CS is usually arranged to be programmed on the basis of a minimum stimulation-frequency value adopted when it is detected that the wearer is at rest (in the present case the condition in which A,B,C,D, = 0,0,0,0).

This minimum frequency is increased when the signal of the movement sensor (the accelerometer ACC in the present case) indicates activity.

As already indicated, and as will be explained further below, the currently-preferred embodiment of the solution according to the invention enables at least three situations or conditions of activity to be identified (that is, distinguished and discriminated from any errors and/or artifacts ), that is:
- a first condition, corresponding to normal ambulation by the patient (walking),
- a second condition corresponding to running by the wearer, and
- a third condition corresponding to descending ambulation (going down stairs, descending a slope, etc.) by the wearer.

It is possible, for example, to arrange for these three different conditions to correspond to the generation, by the unit GS, of stimulation signals with three different frequencies, a low frequency, a medium frequency, and a high frequency, according to whether normal walking, descent, or running by the wearer are detected, respectively. Naturally, changing from one frequency to the other will take place gradually with suitable time constants.

It is, however, possible to operate differently, for example, by arranging for two or more of the frequencies indicated above (by way of indication, those adopted for walking and descending) in fact to be the same and/or to alter solely the time constants, providing only a maximum stimulation frequency in addition to the minimum, rest frequency, and arranging for the frequency adopted at the time in question to fluctuate between these two values by time constants.

It will be appreciated that, whatever strategy is adopted for the selective modification of the stimulation frequency by the unit CS, the solution described permits the generation of an electrical stimulation signal the frequency of which is related to the wearer's pace.

In any case, it is stressed that the invention relates primarily to the criteria adopted to distinguish and discriminate between the various conditions rather than to the stimulation strategies consequently adopted, which may be widely varied.

With reference once more to the possible proposed layout, after the values of the variables A,B,C,D have been generated, the steps detected by the two, low- and high-frequency components are used to calculate the repetition period or step period T; in configurations in which only the low frequency is present, that is, when A,B = 1,1 and D = 0, the period is automatically computed on that component; if, on the other hand, the high frequency is present, that is C,D = 1,1, irrespective of A and B, then the period is computed on the high frequency, excluding the low frequency since the measurement permits greater precision. With regard to configurations such as A,B,C,D = 1,0,0,0-1,0,1,0-0,0,1,0, depending upon the algorithm programmed, the control unit CS may or may not decide to increase the stimulation frequency up to a limit equal to 10 beats above the basic frequency programmed, since either mechanical interference or movements of the patient not necessarily connected with ambulation may be involved.

For stimulation frequency values greater than 10 beats above the basic frequency programmed, these configurations of the parameters A,B,C,D are considered to be artifacts and are treated in the same way as a total absence of signal, that is, as the configuration A,B,C,D, = 0,0,0,0. The unit CS is thus in fact unaffected by these artifacts.

The measurement of the period of the step, regardless of its type, is carried out by a counter CT (the period counter T - shown in Figure 4) in combination with a latch store LATCH which holds the result of the measurement effected by the counter. For each step pulse which comes from the logic unit composed of AND1, AND2, and OR1, the counter first of all saves in the latch store LATCH the result of the last measurement executed and immediately afterwards starts a new measurement using a clock signal, for example, of 1 ms, which comes from the control unit of the pacemaker and in any case can be programmed in dependence on the desired resolution. The measurement of the step period is carried out in the low-frequency chain only if the high-frequency signal is not present; a 500 ms retriggerable monostable unit M7 controls the priority of the steps connected with the high frequency over those relating to the low frequency by means of the logic gates AND1 and AND2.

The control unit CS controls the reading of the periods measured by the counter CT (which, in practice, are indicative of the principal periodicity of the movement signal generated by the movement sensor ACC). These periods are stored in the latch store LATCH in accordance with its own timing linked with the leading edges of the signals output by OR1 and are hence indicative of the steps detected; immediately after it has stored the last step period, the latch store LATCH sends to the control unit CS the signal "datum ready" to indicate the availability of the calculated period value at its outputs. After each reading of the period T and of the variables A,B,C,D, the control unit CS zeroes both these variables and the latch store LATCH.

Naturally, the principle of the invention remaining the same, the details of construction and forms of embodiment may be varied widely with respect to those described and illustrated, without thereby departing from the scope of the present invention. This applies in particular to the logic for generating and processing the variables A,B,C,D; complementary generating logic could, for example, be used (test passed "0", otherwise "1" ) with a corresponding modification of the relative interpretation logic.

## Claims

1. A heart pacemaker for stimulating a wearer's heart (H) by means of an electrical stimulation signal having a selectively variable frequency, the pacemaker comprising:
- a movement sensor (ACC) which can generate a movement signal indicative of the wearer's activity in use, and
- a stimulation-control unit (CS) which is sensitive to the movement signal and can selectively modify the frequency of the stimulation signal in dependence on the movement signal, and
- processing means (200, 300, 400, 500, 600, 700, V) which are sensitive to the movement signal and can generate, from the movement signal, further signals indicative, respectively, of the frequency, and of the type of impact on the ground, of the wearer's step; the control unit (CS) being able to identify (T) respective different conditions of ambulation by the wearer from the further signals and selectively to modify the frequency of the stimulation signal in dependence on the condition of ambulation identified (A,B,C,D),
- filtering means (200, 300) which are supplied with the movement signal and can generate therefrom a first, low-frequency signal and a second, high-frequency signal, indicative of the inertial and vibratory components of the movement signal, respectively,
**characterized in that** the processing means comprise:
- for each of the first and second signals, respective first comparator means (400, 500) sensitive to the duration of the respective first or second signal and having respective duration thresholds in order to carry out respective duration comparisons on the first and second signals, as well as respective second comparator means (600; 700) sensitive to the repetition period of the respective first or second signal and having respective repetition-period thresholds in order to carry out respective repetition-period comparisons on the first and second signals, the first (400, 500) and second (600, 700) comparator means generating respective logic signals (A,B,C,D) which adopt different logic values according to the results of the respective duration and repetition comparisons, and **in that** the stimulation-control unit (CS) selectively modifies the frequency of the stimulation signal in dependence on various combinations of the values of the logic signals (A,B,C,D).

2. A pacemaker according to Claim 1, **characterized in that** the movement sensor (ACC) is associated with the pacemaker in a relationship of mechanical continuity therewith.

3. A pacemaker according to Claim 1 or Claim 2, **characterized in that** the movement sensor (ACC) is fitted on a wall (W) of the pacemaker.

4. A pacemaker according to any one of Claims 1 to 3, **characterized in that** the movement sensor (ACC) is mounted on the pacemaker with the interposition of electrically-insulating and substantially rigid elements (EI).

5. A pacemaker according to any one of Claims 1 to 4, **characterized in that** the movement sensor (ACC) is mounted inside the pacemaker.

6. A pacemaker according to any one of the preceding Claims, **characterized in that** the movement sensor (ACC) is an accelerometric sensor.

7. A pacemaker according to any one of the preceding claims, **characterized in that** the movement signal has a frequential content between about 0.1 and about 100 Hz.

8. A pacemaker according to Claim 1, **characterized in that** the filtering means (200) have a first pass-band such that the first signal has a frequency content between about 1 and about 5 Hz.

9. A pacemaker according to either of Claims 1 and 8, **characterized in that** the filtering means have a second pass-band (300) such that the second signal has a frequency content between about 20 and about 50 Hz.

10. A pacemaker according to any one of Claims 1, 8 or 9, **characterized in that** the first comparator means have a duration threshold of about 10 ms for the first signal.

11. A pacemaker according to any one of Claims 1 or 8 to 10, **characterized in that** the first comparator means have a duration threshold of about 5 ms for the second signal.

12. A pacemaker according to any one of Claims 1 or 8 to 11, **characterized in that** the second comparator means have a repetition-period threshold of about 380 ms for the first signal.

13. A pacemaker according to any one of Claims 1 or 8 to 12, **characterized in that** the second comparator means have a repetition-period threshold of about 300 ms for the second signal.

14. A pacemaker according to any one of Claims 1 or 8 to 13, **characterized in that** squaring circuits (CBF1, CBF2, OR1-BF; CAF1, CAF2, OR2-AF) are interposed between the filtering means (200, 300) and the first and second comparator means (400; 500, 600; 700) for converting the first and second signals into logic signals so that the duration and the repetition period subjected to comparison are identified by the first and second signals remaining at a predetermined logic level.

15. A pacemaker according to any one of Claims 1 or 8 to 14, **characterized in that** the first comparator means (400, 500) have respective associated multiple-pulse exclusion circuits (M3, M4) in order to perform the duration comparison on a single pulse of the respective first or second signal, respectively.

16. A pacemaker according to any one of Claims 1 or 8 to 15, **characterized in that** the stimulation-control unit (CS) is sensitive to:
- a first combination (0,0,0,0) of the respective logic signals (A,B,C,D) which is indicative of a substantial absence of the first and second signals, the stimulation-control unit (CS) generating an electrical stimulation signal with a minimum stimulation frequency in the presence of this first combination, and
- at least one further combination of the respective logic signals (A,B,C,D) in the presence of which the stimulation-control unit (CS) generates an electrical stimulation signal with a frequency greater than the minimum stimulation frequency.

17. A pacemaker according to Claim 16, **characterized in that** the stimulation-control unit (CS) permits at least:
- a first operating condition for the generation of the stimulation signal in conditions in which the wearer is walking normally,
- a second operating condition for the generation of the stimulation signal in conditions in which the wearer is running, and
- a third operating condition for the generation of the stimulation signal in conditions in which the wearer is descending,
and **in that** the stimulation-control unit (CS) recognizes:
- at least a first further combination (1,1,0,0; 1,1,1,0) of the respective logic signals (A,B,C,D) indicative of the fact that the first signal has a duration and a repetition period which reach the respective duration and repetition-period thresholds of the first (400) and second (600) comparator means, whereas the second signal does not reach at least one of the duration threshold and the repetition-period threshold of the first (500) and second (700) comparator means, the stimulation-control unit (CS) adopting the first operating condition upon detection of the at least one further combination, and
- at least a second further combination (0,0,1,1; 1,0,1,1; 1,1,1,1) of the respective logic signals (A,B,C,D) indicative of the fact that the second signal has a duration and a repetition period which reach the respective duration and repetition-period thresholds of the respective first (500) and second (700) comparator means, irrespective of the duration and of the repetition period of the first signal, the stimulation control unit (CS) adopting one of the second and third operating conditions upon detection of the at least one second further combination.

18. A pacemaker according to Claim 17, **characterized in that** it also comprises detector means (V) sensitive to a principal periodicity (T) of the movement signal which is indicative of the period of the wearer's step, the detector means (V) being able to discriminate as to whether the step period value is below or above a step threshold, respectively, and **in that**, upon detection of the at least one second further combination, the stimulation-control unit (CS) adopts the second or third operating condition, respectively, according to whether the step period is below or above the step threshold.

19. A pacemaker according to Claim 18, **characterized in that** the step threshold is equal to about 450 ms.

20. A pacemaker according to any one of Claims 16 to 19, **characterized in that**, in the presence of the at least one further combination of the respective logic signals (A, B, C, D), the stimulation-control unit (CS) generates an electrical stimulation signal with a frequency correlated to the wearer's step frequency.

21. A pacemaker according to any one of preceding Claims 1 or 8 to 20, **characterized in that** the stimulation-control unit (CS) is unaffected by least some combinations of the respective logic signals (A,B,C,D), the combinations by which the stimulation-control unit (CS) is unaffected being indicative of erroneous operating conditions of the pacemaker.

22. A pacemaker according to any one of Claims 1 or 8 to 22, **characterized in that** the stimulation-control unit (CS) recognizes as indicative of detection artifacts of the movement signal combinations of the logic signals (A,B,C,D) corresponding to the fact that the first and second signals have durations which reach the respective duration thresholds of the first (400, 500) comparator means, whereas the first and second signals have repetition periods which do not reach the respective repetition-period thresholds of the second (600, 700) comparator means, and **in that**, in the presence of detection artifacts, the stimulation-control unit (CS) generates the stimulation signal at a predetermined frequency.

23. A pacemaker according to Claim 16 and Claim 22, **characterized in that** the predetermined frequency is greater than the minimum stimulation frequency.

## Patentansprüche

1. Herzschrittmacher zum Stimulieren des Herzens (H) eines Trägers mittels eines elektrischen Stimulationssignals, welches eine selektiv variable Frequenz aufweist, wobei der Schrittmacher umfasst:
- einen Bewegungssensor (ACC), welcher ein Bewegungssignal generieren kann, das kennzeichnend für die Aktivität des Trägers bei der Benutzung ist, und
- eine Stimulierungs-Steuereinheit (CS), welche sensitiv auf das Bewegungssignal reagiert und selektiv die Frequenz des Stimulierungssignals in Abhängigkeit des Bewegungssignals modifizieren kann, und
- Verarbeitungsmittel (200, 300, 400, 500, 600, 700, V), welche sensitiv auf das Bewegungssignal reagieren und aus dem Bewegungssignal weitere Signale generieren können, welche jeweils kennzeichnend für die Frequenz und den Typ des Drucks auf den Boden durch den Schritt des Trägers sind, wobei die Steuereinheit (CS) in der Lage ist, jeweilig unterschiedliche Zustände des Ganges durch den Träger (T) aus den weiteren Signalen zu identifizieren und die Frequenz des Stimulierungssignals in Abhängigkeit von dem Zustand des Ganges, der identifiziert wurde (A, B, C, D) zu modifizieren,
- Filtermittel (200, 300), welche mit dem Bewegungssignal zugeführt werden, und daraus ein erstes niedrigfrequentes Signal und ein zweites hochfrequentes Signal generieren können, welches jeweils indikativ für die Trägheits- und Vibrationskomponenten des Bewegungssignals sind,
**dadurch gekennzeichnet, dass** die Verarbeitungsmittel umfassen:
- für jedes der ersten und zweiten Signale jeweilige erste Komparatormittel (400, 500), die sensitiv für die Dauer des jeweiligen ersten oder zweiten Signals sind und welche jeweilige Dauer-Grenzwerte aufweisen, um jeweilige Dauervergleiche der ersten und zweiten Signale auszuführen, sowie jeweilige zweite Komparatormittel (600; 700), die sensitiv für die Wiederholungsperiode des jeweiligen ersten oder zweiten Signals sind und welche jeweilige Wiederholungsperioden-Grenzwerte aufweisen, um jeweilige Wiederholungsperiodenvergleiche der ersten und zweiten Signale durchzuführen, wobei die ersten (400, 500) und zweiten (600, 700) Komparatormittel jeweilige Logiksignale (A, B, C, D) erzeugen, welche verschiedene logische Werte gemäß den Ergebnissen der jeweiligen Dauer- und Wiederholungsvergleiche annehmen und, dass die Stimulierungssteuereinheit (CS) selektiv die Frequenz des Stimulierungssignals in Abhängigkeit von verschiedenen Kombinationen der Werte der logischen Signale (A, B, C, D) modifiziert.

2. Schrittmacher gemäß Anspruch 1, **dadurch gekennzeichnet, dass** der Bewegungssensor (ACC) mit dem Schrittmacher in einer Beziehung einer mechanischen Kontinuität damit assoziiert ist.

3. Schrittmacher gemäß Anspruch 1 oder 2, **dadurch gekennzeichnet, dass** der Bewegungssensor (ACC) an einer Wand (W) des Schrittmachers angebracht ist.

4. Schrittmacher gemäß irgendeinem der Ansprüche 1 bis 3, **dadurch gekennzeichnet, dass** der Bewegungssensor (ACC) auf dem Schrittmacher montiert ist mit der Zwischenschaltung von elektrisch isolierenden und im Wesentlichen starren Elementen (EI).

5. Schrittmacher gemäß irgendeinem der Ansprüche 1 bis 4, **dadurch gekennzeichnet, dass** der Bewegungssensor (ACC) im Inneren des Schrittmachers montiert ist.

6. Schrittmacher gemäß irgendeinem der vorhergehenden Ansprüche, **dadurch gekennzeichnet, dass** der Bewegungssensor (ACC) ein akzelerometrischer Sensor ist.

7. Schrittmacher gemäß irgendeinem der vorhergehenden Ansprüche, **dadurch gekennzeichnet, dass** das Bewegungssignal einen Frequenzgehalt zwischen ungefähr 0,1 und ungefähr 100 Hz aufweist.

8. Schrittmacher gemäß Anspruch 1, **dadurch gekennzeichnet, dass** die Filtermittel (200) einen ersten Durchlassbereich aufweisen, derartig, dass das erste Signal einen Frequenzgehalt zwischen ungefähr 1 und 5 Hz aufweist.

9. Schrittmacher gemäß Anspruch 1 oder 8, **dadurch gekennzeichnet, dass** die Filtermittel einen zweiten Durchlassbereich (300) aufweisen, derartig, dass das zweite Signal einen Frequenzgehalt zwischen ungefähr 20 und ungefähr 50 Hz aufweist.

10. Schrittmacher gemäß irgendeinem der Ansprüche 1, 8 oder 9, **dadurch gekennzeichnet, dass** die ersten Komparatormittel einen Dauer-Grenzwert von ungefähr 10 ms für das erste Signal aufweisen.

11. Schrittmacher gemäß irgendeinem der Ansprüche 1 oder 8 bis 10, **dadurch gekennzeichnet, dass** die ersten Komparatormittel einen Dauer-Grenzwert von ungefähr 5 ms für das zweite Signal aufweisen.

12. Schrittmacher gemäß irgendeinem der Ansprüche 1 oder 8 bis 11, **dadurch gekennzeichnet, dass** die zweiten Komparatormittel einen Wiederholungsperioden-Grenzwert von ungefähr 380 ms für das erste Signal aufweisen.

13. Schrittmacher gemäß irgendeinem der Ansprüche 1 oder 8 bis 12, **dadurch gekennzeichnet, dass** die zweiten Komparatormittel einen Wiederholungsperioden-Grenzwert von ungefähr 300 ms für das zweite Signal aufweisen.

14. Schrittmacher gemäß irgendeinem der Ansprüche 1 oder 8 bis 13, **dadurch gekennzeichnet, dass** die Quadrierschaltungen (CBF1, CBF2, OR1-BF; CAF1; CAF2, OR2-AF) zwischen den Filtermitteln (200, 300) und den ersten und zweiten Komparatormittel (400; 500, 600; 700) zum Konvertieren der ersten und zweiten Signale in logische Signale zwischengeschaltet sind, so dass die Dauer und die Wiederholungsperiode, die dem Vergleich unterliegen, durch die ersten und zweiten Signale identifiziert werden, die an einem vorbestimmten logischen Niveau verbleiben.

15. Schrittmacher gemäß irgendeinem der Ansprüche 1 oder 8 bis 14, **dadurch gekennzeichnet, dass** die ersten Komparatormittel (400, 500) jeweilig assoziierte Multi-Impuls-Ausschaltschaltungen (M3, M4) aufweisen, um den Dauervergleich in einem einzigen Impuls des jeweiligen ersten oder zweiten Signals durchzuführen.

16. Schrittmacher gemäß irgendeinem der Ansprüche 1 oder 8 bis 15, **dadurch gekennzeichnet, dass** die Stimulierungssteuereinheit (CS) sensitiv ist gegenüber:
- einer ersten Kombination (0, 0, 0, 0) der jeweiligen logischen Signale (A, B, C, D), welche indikativ für eine wesentliche Abwesenheit der ersten und zweiten Signale ist, wobei die Stimulierungssteuereinheit (CS) ein elektrisches Stimulierungssignal mit einer Minimum-Stimulierungsfrequenz in Anwesenheit dieser ersten Kombination generiert, und
- zumindest eine weitere Kombination der jeweiligen logischen Signale (A, B, C, D) in der Anwesenheit welcher die Stimulierungssteuereinheit (CS) ein elektrisches Stimulierungssignal mit einer Frequenz generiert, die größer ist als die Minimum-Stimulierungsfrequenz.

17. Schrittmacher gemäß Anspruch 16, **dadurch gekennzeichnet, dass** die Stimulierungssteuereinheit (CS) zumindest erlaubt:
- einen ersten Betriebszustand für die Erzeugung des Stimulierungssignals in Zuständen, bei welchen der Träger normal geht,
- einen zweiten Betriebszustand für die Erzeugung des Stimulierungssignals in Zuständen, bei welchen der Träger läuft, und
- einen dritten Betriebszustand zum Erzeugen des Stimulierungssignals in Zuständen, in welchen der Träger absteigend ist,
und dadurch, dass die Stimulierungssteuereinheit (CS) erkennt:
- zumindest eine erste weitere Kombination (1, 1, 0, 0; 1, 1, 1, 0) der jeweiligen logischen Signale (A, B, C, D), die indikativ für die Tatsache sind, dass das erste Signal eine Dauer und eine Wiederholungsperiode aufweist, welche die jeweiligen Dauer- und Wiederholungsperioden-Grenzwerte der ersten (400) und zweiten (600) Komparatormittel erreicht, wobei das zweite Signal nicht zumindest entweder den Dauer-Grenzwert oder den Wiederholungsperioden-Grenzwert der ersten (500) und zweiten (700) Komparatormittel erreicht, wobei die Stimulierungssteuereinheit (CS) den ersten Betriebszustand auf eine Detektion der zumindest einen weiteren Kombination hin annimmt, und
- zumindest eine zweite weitere Kombination (0, 0, 1, 1; 1, 0, 1, 1; 1, 1, 1, 1) der jeweiligen logischen Signale (A, B, C, D), die indikativ für die Tatsache ist, dass das zweite Signal eine Dauer und eine Wiederholungsperiode aufweist, welche die jeweiligen Dauer- und Wiederholungsperioden-Grenzwerte der jeweiligen ersten (500) und zweiten (700) Komparatormittel erreichen, ungeachtet der Dauer und der Wiederholungsperiode des ersten Signals, wobei die Stimulierungssteuereinheit (CS) entweder den zweiten oder dritten Betriebszustand annimmt auf eine Detektion der zumindest zweiten weiteren Kombination hin.

18. Schrittmacher gemäß Anspruch 17, **dadurch gekennzeichnet, dass** er auch Detektiermittel (V) umfasst, die sensitiv gegenüber einer Grundperiodizität (T) des Bewegungssignals sind, welches indikativ für die Dauer des Schrittes des Trägers ist, wobei die Detektiermittel (V) in der Lage sind, zu unterscheiden, ob der Schrittperiodenwert unterhalb oder oberhalb eines Schritt-Grenzwerts ist bzw. dadurch, dass auf eine Detektion der zumindest einen zweiten weiteren Kombination hin die Stimulierungssteuereinheit (CS) den zweiten bzw. dritten Betriebszustand annimmt, entsprechend dazu, ob die Schrittperiode unterhalb bzw. oberhalb des Schritt-Grenzwertes ist.

19. Schrittmacher gemäß Anspruch 18, **dadurch gekennzeichnet, dass** der Schritt-Grenzwert gleich ungefähr 450 ms ist.

20. Schrittmacher gemäß irgendeinem der Ansprüche 16 bis 19, **dadurch gekennzeichnet, dass** beim Vorhandensein von zumindest einer weiteren Kombination der jeweiligen logischen Signale (A, B, C, D) die Stimulierungssteuereinheit (CS) ein elektrisches Stimulierungssignal mit einer Frequenz erzeugt, die mit der Schrittfrequenz des Trägers korreliert ist.

21. Schrittmacher gemäß irgendeinem der vorhergehenden Ansprüche 1 oder 8 bis 20, **dadurch gekennzeichnet, dass** die Stimulierungssteuereinheit (CS) unbeeinflusst durch zumindest einige Kombinationen der jeweiligen logischen Signale (A, B, C, D) verbleibt, wobei die Kombinationen, durch welche die Stimulierungssteuereinheit (CS) unbeeinflusst verbleibt, indikativ für fehlerhafte Betriebszustände des Schrittmachers sind.

22. Schrittmacher gemäß irgendeinem der Ansprüche 1 oder 8 bis 21, **dadurch gekennzeichnet, dass** die Stimulierungssteuereinheit (CS) als indikativ für Detektion Artefakte der Bewegungssignalkombinationen der logischen Signale (A, B, C, D) entsprechend der Tatsache erkennt, dass die ersten und zweiten Signale Dauern aufweisen, welche die jeweiligen Dauer-Grenzwerte der ersten (400, 500) Komparatormittel erreichen, wobei die ersten und zweiten Signale Wiederholungsperioden aufweisen, welche nicht die jeweiligen Wiederholungsperioden-Grenzwerte der zweiten (600, 700) Komparatormittel erreichen und, dass beim Vorhandensein von Detektionsartefakten die Stimulierungssteuereinheit (CS) das Stimulierungssignal mit einer vorbestimmten Frequenz erzeugt.

23. Schrittmacher gemäß Anspruch 16 und 22, **dadurch gekennzeichnet, dass** die vorbestimmte Frequenz größer ist als die minimale Stimulierungsfrequenz.

## Revendications

1. Un stimulateur cardiaque pour stimuler le coeur (H) d'un porteur au moyen d'un signal de stimulation électrique présentant une fréquence sélectivement variable, le stimulateur comprenant :
- un capteur de mouvement (ACC) qui peut produire un signal de mouvement représentatif de l'activité en cours du porteur,
- une unité de commande de stimulation (CS) qui est sensible au signal de mouvement et peut sélectivement modifier la fréquence du signal de stimulation selon le signal de mouvement, et
- des moyens de traitement (200, 300, 400, 500, 600, V) qui sont sensibles au signal de mouvement et peuvent produire, à partir du signal de mouvement, d'autres signaux représentatifs, respectivement, de la fréquence et du type d'impact sur le sol des pas du porteur ; l'unité de commande (CS) étant capable d'identifier (T) différentes conditions ambulatoires respectives du porteur à partir des autres signaux et de sélectivement modifier la fréquence du signal de stimulation selon la condition ambulatoire identifiée (A, B, C, D),
- des moyens de filtrage (200, 300) qui sont alimentés par le signal de mouvement et peuvent générer à partir de celui-ci un premier signal, à basse fréquence, et un second signal, à haute fréquence, représentatifs des composantes inertielle et vibratoire du signal de mouvement, respectivement,
**caractérisé en ce que** les moyens de traitement comprennent :
pour chacun des premier et second signal, des premiers moyens comparateurs respectifs (400, 500) sensibles à la durée des premier ou second signal respectifs et possédant des seuils de durée respectifs afin d'opérer des comparaisons de durées respectives sur le premier et le second signal, ainsi que des seconds moyens comparateurs respectifs (600, 700) sensibles à la période de répétition des premier ou second signal respectifs et possédant des seuils de période de répétition respectifs afin d'opérer des comparaisons de périodes de répétition respectives sur le premier et le second signal, les premiers (400, 500) et second (600, 700) moyens comparateurs produisant des signaux logiques respectifs (A, B, C, D) qui prennent des valeurs logiques différentes en fonction des résultats des comparaisons respectives de durées et de périodes de répétition, et **en ce que** l'unité de commande de stimulation (CS) modifie sélectivement la fréquence du signal de stimulation selon les diverses combinaisons des valeurs des signaux logiques.

2. Un stimulateur selon la revendication 1, **caractérisé en ce que** le capteur de mouvement (ACC) est associé au stimulateur en une relation de continuité mécanique avec celui-ci.

3. Un stimulateur selon la revendication 1 ou la revendication 2, **caractérisé en ce que** le capteur de mouvement (ACC) est disposé sur une paroi (W) du stimulateur.

4. Un stimulateur selon l'une des revendications 1 à 3, **caractérisé en ce que** le capteur de mouvement (ACC) est monté sur le stimulateur avec interposition d'éléments électriquement isolants et substantiellement rigides (EI).

5. Un stimulateur selon l'une des revendications 1 à 4, **caractérisé en ce que** le capteur de mouvement (ACC) est monté à l'intérieur du stimulateur.

6. Un stimulateur selon l'une quelconque des revendications précédentes, **caractérisé en ce que** le capteur de mouvement (ACC) est un capteur accélérométrique.

7. Un stimulateur selon l'une quelconque des revendications précédentes, **caractérisé en ce que** le signal de mouvement possède un contenu fréquentiel entre environ 0,1 et environ 100 Hz.

8. Un stimulateur selon la revendication 1, **caractérisé en ce que** les moyens de filtrage (200) possèdent un premier passe-bande tel que le premier signal possède un contenu fréquentiel entre environ 1 et environ 5 Hz.

9. Un stimulateur selon l'une ou l'autre des revendications 1 et 8, **caractérisé en ce que** les moyens de filtrage possèdent un second passe-bande (300) tel que le second signal possède un contenu fréquentiel entre environ 20 et environ 50 Hz.

10. Un stimulateur selon l'une quelconque des revendications 1 ou 8 à 9, **caractérisé en ce que** les premiers moyens comparateurs possèdent un seuil de durée d'environ 10 ms pour le premier signal.

11. Un stimulateur selon l'une quelconque des revendications 1 ou 8 à 10, **caractérisé en ce que** les premiers moyens comparateurs possèdent un seuil de durée d'environ 5 ms pour le premier signal.

12. Un stimulateur selon l'une quelconque des revendications 1 ou 8 à 11, **caractérisé en ce que** les seconds moyens comparateurs possèdent un seuil de période de répétition d'environ 380 ms pour le premier signal.

13. Un stimulateur selon l'une quelconque des revendications 1 ou 8 à 12, **caractérisé en ce que** les seconds moyens comparateurs possèdent un seuil de période de répétition d'environ 300 ms pour le premier signal.

14. Un stimulateur selon l'une quelconque des revendications 1 ou 8 à 13, **caractérisé en ce que** des circuits de mise au carré (CBF1, CBF2, OR1-BF ; CAF1, CAF2, OR2-AF) sont interposés entre les moyens de filtrage (200, 300) et les premiers et seconds moyens comparateurs (400, 500 ; 600, 700) pour convertir les premier et second signal en signaux logiques de manière à pouvoir identifier la durée et la période de répétition soumises à comparaison par le fit que les premier et second signaux restent à un niveau logique prédéterminé.

15. Un stimulateur selon l'une quelconque des revendications 1 ou 8 à 14, **caractérisé en ce que** les premiers moyens comparateurs (400, 500) possèdent des circuits d'exclusion d'impulsions multiples associés (M3, M4) afin d'opérer la comparaison de durée sur une impulsion unique du premier et second signal, respectivement.

16. Un stimulateur selon l'une quelconque des revendications 1 ou 8 à 15, **caractérisé en ce que** l'unité de commande de stimulation (CS) est sensible à :
- une première combinaison (0, 0, 0, 0) des signaux logiques respectifs (A, B, C, D) qui est représentative d'une absence substantielle des premier et second signal, l'unité de commande de stimulation (CS) produisant un signal de stimulation électrique avec une fréquence minimale de stimulation en présence de cette première combinaison, et
- au moins une autre combinaison des signaux logiques respectifs (A, B, C, D) en présence de laquelle l'unité de commande de stimulation (CS) produit un signal de stimulation électrique avec une fréquence supérieure à la fréquence minimale de stimulation.

17. Un stimulateur selon la revendication 16, **caractérisé en ce que** l'unité de commande de stimulation (CS) permet au moins :
- un premier état de fonctionnement pour la production du signal de stimulation dans des états où le porteur est en train de marcher normalement,
- une second état de fonctionnement pour la production du signal de stimulation dans des états où le porteur est en train de courir,
- un troisième état de fonctionnement pour la production du signal de stimulation dans des états où le porteur est en train de descendre,
et **en ce que** l'unité de commande de stimulation (CS) reconnaît :
- au moins une première autre combinaison (1, 1, 0, 0 ; 1, 1, 1, 0) des signaux logiques respectifs (A, B, C, D) représentative du fait que le premier signal présente une durée et une période de répétition qui atteignent les seuils de durée et de période de répétition respectifs des premiers (400) et des seconds (600) moyens comparateurs, alors que le second signal n'atteint pas au moins l'un du seuil de durée et du seuil de période de répétition des premiers (500) et des seconds (700) moyens comparateurs, l'unité de commande de stimulation (CS) prenant le premier état de fonctionnement à détection de ladite au moins une autre combinaison, et
- au moins une seconde autre combinaison (0, 0, 1, 1 ; 1, 0, 1, 1 ; 1, 1, 1, 1) des signaux logiques respectifs (A, B, C, D) représentative du fait que le second signal présente une durée et une période de répétition qui atteignent les seuils de durée et de période de répétition respectifs des premiers (500) et des seconds (700) moyens comparateurs, indépendamment de la durée et de la période de répétition du premier signal, l'unité de commande de stimulation (CS) prenant l'un des second et troisième états de fonctionnement à détection de ladite au moins seconde autre combinaison.

18. Un stimulateur selon la revendication 17, **caractérisé en ce qu'**il comprend également des moyens détecteurs (V) sensibles à une périodicité principale (T) du signal de mouvement qui est représentative de la période des pas du porteur, les moyens détecteurs étant capables de discriminer selon que la valeur de la période des pas est au-dessous ou au-dessus d'un seuil de pas, respectivement, et **en ce que**, à détection de ladite au moins seconde autre combinaison, l'unité de commande de stimulation (CS) prend le second ou le troisième état de fonctionnement, respectivement, selon que la période des pas est au-dessous ou au-dessus du seuil de pas.

19. Un stimulateur selon la revendication 18, **caractérisé en ce que** le seuil de pas est égal à environ 450 ms.

20. Un stimulateur selon l'une quelconque des revendications 16 à 19, **caractérisé en ce que**, en présence de ladite au moins une autre combinaison des signaux logiques respectifs (A, B, C, D), l'unité de commande de stimulation (CS) produit un signal de stimulation électrique avec une fréquence corrélée à la fréquence des pas du porteur.

21. Un stimulateur selon l'une quelconque des revendications 1 ou 8 à 20, **caractérisé en ce que** l'unité de commande de stimulation (CS) n'est pas affectée par au moins certaines combinaisons des signaux logiques respectifs (A, B, C, D), les combinaisons par lesquelles l'unité de commande de stimulation (CS) n'est pas affectée étant représentatives d'états de fonctionnement erronés du stimulateur.

22. Un stimulateur selon l'une quelconque des revendications 1 ou 8 à 22, **caractérisé en ce que** l'unité de commande de stimulation (CS) reconnaît comme représentatives d'artefacts de détection du signal de mouvement des combinaisons des signaux logiques (A, B, C, D) correspondant au fait que les premier et second signal ont des durées qui atteignent les seuils de durée respectifs des premiers (400, 500) moyens comparateurs, alors que les premier et second signal ont des périodes de répétition qui n'atteignent pas les seuils respectifs de période de répétition des seconds (600, 700) moyens comparateurs, et **en ce que**, en présence d'artefacts de détection, l'unité de commande de stimulation (CS) produit le signal de stimulation à une fréquence prédéterminée.

23. Un stimulateur selon la revendication 16 et la revendication 22, **caractérisé en ce que** la fréquence prédéterminée est supérieure à la fréquence minimale de stimulation.
